# EUROPÄISCHE PATENTSCHRIFT

(11) **EP 2 374 506 B1**
(45) Veröffentlichungstag und Bekanntmachung des Hinweises auf die Patenterteilung: **15.05.2019**
(21) Anmeldenummer: 11156003.3
(22) Anmeldetag: 25.02.2011
(51) Int. Cl.: A61N 5/10

(54) **Partikeltherapieanlage**
Particle therapy system
Installation de thérapie à particules

(30) Priorität: 07.04.2010 DE 102010014002
(43) Veröffentlichungstag der Anmeldung: 12.10.2011
(73) Patentinhaber: Varian Medical Systems Particle Therapy GmbH, 53842 Troisdorf (DE)
(72) Erfinder: Bräuer, Martin, 90402, Nürnberg (DE)
(74) Vertreter: Sedlacek, Anton

(56) Entgegenhaltungen:
- WO-A1-03/092340
- WO-A1-2009/056165
- WO-A1-2010/149740
- WO-A2-00/40064
- WO-A2-03/069634
- DE-A1- 19 907 138
- JP-A- 11 329 800
- US-A1- 2009 236 545
- US-B2- 6 873 123
- SATO ET AL: "Dynamic intensity control system with RF-knockout slow-extraction in the HIMAC synchrotron", NUCLEAR INSTRUMENTS & METHODS IN PHYSICS RESEARCH, SECTION - A:ACCELERATORS, SPECTROMETERS, DETECTORS AND ASSOCIATED EQUIPMENT, ELSEVIER, AMSTERDAM, NL, Bd. 574, Nr. 2, 6. April 2007 (2007-04-06) , Seiten 226-231, XP022022715, ISSN: 0168-9002, DOI: 10.1016/J.NIMA.2007.01.174
- INANIWA TAKU ET AL: "Optimization for fast-scanning irradiation in particle therapy", MEDICAL PHYSICS, AIP, MELVILLE, NY, US, vol. 34, no. 8, 26 July 2007 (2007-07-26), pages 3302-3311, XP012103531, ISSN: 0094-2405, DOI: 10.1118/1.2754058

## Beschreibung

Die vorliegende Erfindung betrifft eine Partikeltherapieanlage. Partikeltherapieanlagen werden zur Behandlung von Tumoren mit schweren Teilchen, wie zum Beispiel Protonen oder Kohlenstoffionen, verwendet. Dabei wird ein Tumor mit schweren Teilchen, beispielsweise im so genannten Rasterscanverfahren, bestrahlt. Neben Tumoren von Patienten können auch Phantome, z.B. zu Forschungs- oder zu Wartungszwecken, bestrahlt werden. Basis des Rasterscanverfahrens ist, dass die von einer Teilchenerzeugungs- und Beschleunigervorrichtung gelieferte Intensität der Strahlung für jede ISO-Energieschicht eingestellt wird und die auf jeden Rasterpunkt angewendete Dosis in Echtzeit erfasst wird und für jeden Rasterpunkt gewartet wird, bis die geplante Zieldosis erreicht ist. Die Strahlung kann beispielsweise mit Hilfe von schnellen Magneten ein- und ausgeschaltet werden. Da eine minimale Zeit pro Rasterpunkt während der Anwendung der Strahlung auf einen Rasterpunkt notwendig ist, bestimmt diese minimale Zeit für die Anwendung der Bestrahlung eines Rasterpunkts mit einer minimalen Dosis die maximale Strahlintensität, die verwendet werden kann. Dabei muss darüber hinaus berücksichtigt werden, dass die technologischen Schemata zur Extraktion des Teilchenstrahls aus der Teilchenerzeuger- und Beschleunigervorrichtung zu Teilchenstromverläufen führen, welche großen Fluktuationen unterliegen. Typische Schemata zur Extraktion der Teilchenstrahlen sind beispielsweise eine Resonanzextraktion oder eine Extraktion mit einem so genannten Knock-Out-Exciter (KO-Exciter). Die so erzeugten Teilchen- oder Partikelstromverläufe werden durch schnelle Fluktuationen im Bereich einiger Mikrosekunden dominiert und verändert sich darüber hinaus insbesondere zu Beginn der Extraktion zusätzlich in einem Zeitbereich von einigen 10 Millisekunden bis zu einigen Sekunden.

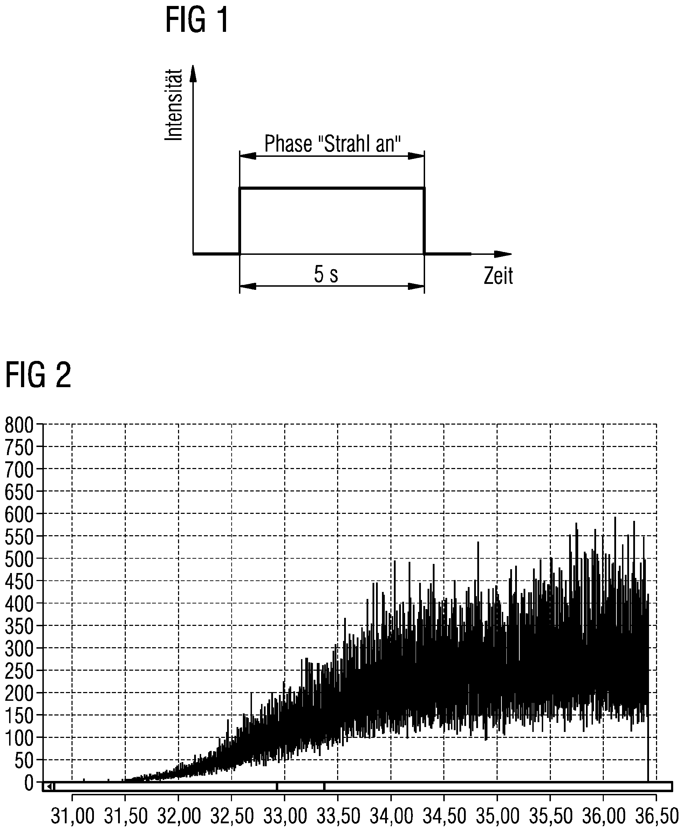

Wünschenswert wäre hingegen, wenn eine Partikelstrahlintensität, das heißt eine Anzahl von Teilchen oder Partikeln pro Zeiteinheit, annähernd wie die in Figur 1 gezeigte Rechteckfunktion verläuft, d.h. zu einem vorbestimmten Zeitpunkt sehr steil auf die gewünschte Intensität ansteigt und nach einer vorbestimmten Zeit von beispielsweise 5 Sekunden wieder sehr steil abfällt. Figur 2 zeigt hingegen einen Intensitätsverlauf eines Partikelstrahls gemäß dem Stand der Technik. Die Erfassung der Intensität erfolgte mit einer Auflösung von 50 ps. Es sind sowohl die langfristigen Fluktuationen wie auch die sehr kurzzeitigen Fluktuationen erkennbar.

In diesem Zusammenhang ist aus der JP-11329800 ein Verfahren für eine ausgehende Strahlung eines geladenen Partikelstrahls und eine Beschleunigungsvorrichtung bekannt. Die Beschleunigungsvörrichtung umfasst eine Vorrichtung zum Aufprägen einer Hochfrequenz, eine Ablenkvorrichtung für eine Ausstrahlung, eine Strommessvorrichtung und eine Rechenmaschine. Die Vorrichtung zum Einprägen der Hochfrequenz erzeugt ein hochfrequentes elektrisches Feld, magnetisches Feld oder elektromagnetisches Feld auf der Grundlage eines Hochfrequenzsignals, um dieses auf einen geladenen Partikelstrom aufzuprägen. Die Ableitvorrichtung strahlt den geladenen Partikelstrahl, welcher zu einer Außenseite einer Resonanzstabilitätsgrenze durch die Vorrichtung zum Einprägen der Hochfrequenz bewegt wird, aus. Die Strommessvorrichtung misst einen Wert eines Stroms des geladenen Partikelstrahls, welcher von der Ableitvorrichtung für eine Ausstrahlung ausgestrahlt wird. Die Berechnungsmaschine bestimmt eine Intensität des Hochfrequenzsignals gemäß dem Wert des von der Strommessvorrichtung gemessenen Stroms.

Die US 6,873,123 B2 betrifft eine Vorrichtung zum Regeln der Intensität eines von einem Teilchenbeschleuniger extrahierten. Die Vorrichtung umfasst einen Vergleicher, welcher einen Unterschied zwischen einem digitalen Signal, welches die an dem Ausgang des Beschleunigers gemessene Strahlintensität darstellt, und einem Einstellpunktwert der Strahlintensität bestimmt, eine Smith-Vorhersagevorrichtung, welche einen korrigierten Wert der Strahlintensität auf der Grundlage der Differenz bestimmt, und eine invertierte Zuordnungstabelle, welche einen Einstellpunktwert für die Zuführung des Lichtbogenstroms der Ionenquelle auf der Grundlage des korrigierten Werts und der Strahlintensität bereitstellt.

Die WO 2009/056165 A1 betrifft einen zirkulären Partikelbeschleuniger, welcher in der Lage ist, den Partikelstrahlstrom zu modulieren, welcher den zirkulären Partikelbeschleuniger verlässt. Der Partikelstrahlbeschleuniger umfasst einen Regler, welcher in der Lage ist, eine Elektrodenspannungsamplitude zu modulieren, indem ein gegebener Einstellpunkt der Stromintensität des Partikelstrahls und der gemessene Wert der Stromintensität des Partikelstrahls verglichen werden.

Die WO 03/069634 A2 betrifft ein Verfahren zum Steuern einer nach dem Rasterscanverfahren arbeitenden Bestrahlungseinrichtung für schwere Ionen oder Protonen mit Strahlenextraktion. Die Strahlenergie, Strahlfokussierung und Strahlintensität wird für jeden Beschleunigerzyklus eingestellt und die Strahlextraktion wird für jeden Beschleunigerzyklus festgelegt.

Der Artikel von Sato et al: "Dynamic Intensity control system with RF-knockout slow-extraction in the HIMAC Synchrotron" in Nuclear Instruments & Methods in Physics Research, Section - A:Accelerators, Spectrometers, Detectors and Associated Equipment, Elsevier, Amsterdam, NL, Bd. 574, Nr. 2, 6. April 2007 (2007-04-06), Seiten 226-231, XP022022715, ISSN: 0168-9002, DOI: 10.1016/J.NIM.A2007.01.174, betrifft ein dynamisches Strahlintensitätssteuersystem, welches für eine Verwendung bei einer dreidimensionalen Pencil-Beam-Scanning-Bestrahlung geeignet ist. Bei dem System wird die Amplitude des RF-Knockout unter Verwendung einer 10kHz-Response gesteuert. Die Amplitudenmodulationsfunktion wird auf der Grundlage eines analytischen eindimensionalen Modells der RF-Knockout-Auskopplung erzeugt. Unter Verwendung einer Rückkopplungssteuerung von Proportional- und Integralsteuerungen ermöglicht dieses System, den Strahlstrom dynamisch in Antwort auf eine Anforderung zu steuern.

Die DE 199 07 138 A1 betrifft ein Verfahren zur Überprüfung der Strahlerzeugungsmittel und der Strahlbeschleunigungsmittels einen Ionenstrahl-Therapiesystems. Das Ionenstrahl-Therapiesystem umfasst eine in einem Strahlführungssystem angeordnete Rasterscaneinrichtung. Eine Überprüfung der Ionensorte, der Ionenstrahlenergie, der Ionenstrahlintensität und der Beschleuniger-Verriegelung sowie der Mittel zum Extraktionsabbnich wird durchgeführt.

WO 00/40064 offenbart ein Verfahren zur Bestrahlung eines Zielgebietes mit einem Partikelstrahl, wobei die Scanngeschwindigkeit und die Partikelstrahlintensität gleichzeitig variiert werden. Aufgrund der starken Fluktuationen der Partikelstrahlintensität, welche sich insbesondere bei der Resonanzextraktion zeigt, wird die Bestrahlungstechnik derzeit derart ausgelegt, dass Bestrahlungsintensitäten mit sehr höher Variabilität akzeptiert werden. Daher werden verhältnismäßig lange Bestrahlungszeiten in Kauf genommen, welche durch die Dosis von Rasterpunkten geringster Dosis dominiert werden. Weiterhin werden, um trotzdem akzeptable Bestrahlungszeiten zu erhalten, die minimalen Dosen pro Rasterpunkt bei der Planung relativ hoch gesetzt. Dadurch können extreme Fälle vermieden werden, bei denen die Dauer der Strahlanwendung wegen der Beschränkung auf die kleine Intensität sehr groß wird. Andererseits werden dadurch unter Umständen unverhältnismäßig hohe Dosen bei Rasterpunkten mit einem geringen Dosisbedarf in Kauf genommen.

Aufgabe der vorliegenden Erfindung ist es daher, den Betrieb einer Partikeltherapieanlage dahingehend zu verbessern, dass Bestrahlungszeiten verkürzt werden und sowohl kleine als auch große Strahlungsdosen an unterschiedlichen Rasterpunkten angewendet werden können.

Eine weitere Aufgabe ist es, Störungen oder zukünftig erforderliche Wartungen an der Partikeltherapieanlage frühzeitig zu erkennen.

Gemäß der vorliegenden Erfindung werden diese Aufgaben durch eine Partikeltherapieanlage nach Anspruch 1 und einen elektronisch lesbaren Datenträger nach Anspruch 7 gelöst. Die abhängigen Ansprüche definieren bevorzugte und vorteilhafte Ausführungsformen der Erfindung.

Gemäß einem Verfahren zum Betreiben einer Partikeltherapieanlage nach Anspruch 1 werden Bestrahlungspartikel erzeugt und beschleunigt und ein Partikelstrahl aus zumindest einem Anteil der erzeugten Bestrahlungspartikel erzeugt. Weiterhin wird automatisch eine Partikelstrahlintensität des Partikelstrahls erfasst. Außerdem werden mehrere Rasterpunkte gemäß einem vorgegebenen Bestrahlungsplan automatisch mit dem Partikelstrahl sequentiell bestrahlt. In Abhängigkeit von eines jeveiligen Bestrehlungsdosis, welche gemäß dem ßestrahlungsplan jedem das Raster pankte Zaglardnet ist, wird jedem Rasterpunkt jeweils ein Sollwert für die Partikelstrahlintensität an diesem Rasterpunkt zugeordnet. Die Partikelstrahlintensität wird automatisch in Abhängigkeit von der erfassten Partikelstrahlintensität und dem jeweils vorgegebenen Sollwert für die Partikelstrahlintensität des zu bestrahlenden Rasterpunkts beeinflusst.

Indem die Partikelstrahlintensität gemessen wird und automatisch in Abhängigkeit von dem vorgegebenen Sollwert beeinflusst oder eingestellt wird, lassen sich Strahlungsintensitätsverläufe als Funktion der Zeit realisieren, welche beispielsweise dem in Fig. 1 gezeigten gewünschten Verlauf mit einem sehr steilen Anstieg, gefolgt von einem konstanten Verlauf und einem schnellen Abfall sehr nahe kommen. Zur automatischen Erfassung der Partikelstrahlintensität können beispielsweise Detektoren verwendet werden, welche in Partikeltherapieanlagen zur Messung der pro Rasterpunkt angewendeten Dosis verwendet werden. Somit müssen keine zusätzlichen Detektoren in den Partikelstrahl eingebracht werden, welche die Strahlqualität und damit das medizinische Behandlungsergebnis beeinträchtigen. Die von den Detektoren gemessene Strahlintensität kann beispielsweise in Echtzeit digital zu einem digitalen Regler gegeben werden. Zur Übertragung der digitalisierten Messsignale können beispielsweise industrieübliche Bussignale verwendet werden. Der Regler kann beispielsweise ein so genannter PID-Regler sein, welcher eine Proportionalregelung, eine integrierende Regelung und eine differenzierende Regelung verwendet. Der PID-Regler ist insbesondere zum Ausregeln von sowohl den schnellen als auch den langsamen Fluktuationen, welche in Fig. 2 gezeigt sind, geeignet. Alternativ können auch andere in dem Stand der Technik bekannte Regler verwendet werden. Neben einer digitalen Übertragung der von den Detektoren erfassten Partikelstrahlintensität kann auch eine analoge Übertragung vorgesehen sein.

Darüber hinaus ist die Strahlintensität nicht mehr durch den Rasterpunkt niedrigster Dosis beschränkt und die Bestrahlungszeit kann erheblich verringert werden. Weiterhin können die minimalen Dosen pro Rasterpunkt in dem Bestrahlungsplan deutlich geringer angesetzt werden. Dadurch kann eine verbesserte Homogenität und Konformität der Bestrahlung erreicht werden und gleichzeitig die Bestrahlungszeit verringert werden. Dies führt zu einem deutlich erhöhten Patientendurchsatz und somit einer effektiveren Nutzung der Partikeltherapieanlage.

Es kann auch vorgesehen sein, dass eine Änderung des Sollwertes während der sequentiellen Bestrahlung der mehreren Rasterpunkte bei einigen Rasterpunkten vorgesehen sein kann. Insbesondere kann eine Änderung des Sollwertes während der Bestrahlung der zu einer ISO-Energieschicht gehörigen Rasterpunkte stattfinden. Auch dadurch ist die Strahlintensität nicht mehr durch den Rasterpunkt niedrigster Dosis in einer ISO-Energieschicht beschränkt und die Bestrahlungszeit kann erheblich verringert werden. Bei der Bestrahlungsplanung ist man flexibler, da die minimalen Dosen pro Rasterpunkt deutlich geringer angesetzt werden können.

Vorzugsweise wird der Partikelstrahl zu einem Partikelstrahlausgang eines Behandlungsplatzes geleitet und die Partikelstrahlintensität an dem Partikelstrahlausgang des Behandlungsplatzes erfasst. Dadurch kann die automatische Beeinflussung der Partikelstrahlintensität derart eingestellt werden, dass an dem Partikelstrahlausgang des Behandlungsplatzes die gewünschte Partikelstrahlintensität bereitgestellt wird, wodurch eine Beeinflussung der Partikelstrahlintensität beim Leiten des Partikelstrahls zu dem Partikelstrahlausgang entsprechend berücksichtigt werden kann.

Vorzugsweise werden die Bestrahlungspartikel mit einem Synchrotron und/oder einem Linearbeschleuniger erzeugt und beschleunigt. Alternativ können die Bestrahlungspartikel auch mit einem Zyklotron erzeugt und beschleunigt werden. Bei einer Verwendung eines Synchrotrons und Linearbeschleunigers kann zur Beeinflussung der Partikelstrahlintensität ein Anteil der Bestrahlungspartikel mit Hilfe eines so genannten Knock-Out-Exciters ausgekoppelt werden. Der Knock-Out-Exciter wird beispielsweise mit Hilfe eines Steuergeräts angesteuert und gibt eine Hochfrequenz einstellbarer Stärke auf den Teilchenstrahl im Beschleuniger. Je höher die Leistung ist, desto mehr Teilchen werden pro Zeit ausgekoppelt. Um einen gewünschten Partikelstrahlintensitätsverlauf zu erhalten, wird in dem Steuergerät des Knock-Out-Exciters die Ausgangsleistung automatisch mit beispielsweise dem zuvor genannten PID-Regler eingestellt. Dadurch entfällt eine üblicherweise notwendige Einstellung von Parametern von Funktionen, welche den zeitlichen Verlauf der Ausgangsleistung des Knock-Out-Exciters für eine Vorsteuerung einstellen. Weiterhin wird durch die höhe zeitliche Konstanz der Partikelstrahlintensität die Anwendung des Partikelstrahls präziser und homogener. Dadurch vereinfachen sich auch Kontroll- und Überwachungssysteme für die Partikelstrahlanwendung, wodurch der Betrieb sicherer wird. Durch die so erreichte optimale und konstante Strahlungsintensität kann die Häufigkeit von Strahlunterbrechungen, so genannten Interlocks, verringert werden, wodurch die Behandlung schneller durchgeführt werden kann und die Partikeltherapieanlage effizienter genutzt werden kann.

Der Partikelstrahl kann wahlweise zu einem von mehreren Behandlungsplätzen geleitet werden. Die Partikelstrahlintensität, insbesondere die applizierte Partikelstrahlintensität, wird an jedem Behandlungsplatz erfasst und es wird automatisch die erfasste Partikelstrahlintensität desjenigen Behandlungsplatzes zur Beeinflussung der Partikelstrahlintensität verwendet, zu welchem der Partikelstrahl aktuell geleitet wird. Indem an jedem Behandlungsplatz die Partikelstrahlintensität erfasst wird und zur Beeinflussung der Partikelstrahlintensität verwendet wird, kann sichergestellt werden, dass an dem Behandlungsplatz, an dem aktuell eine Bestrahlung durchgeführt wird, exakt die gewünschte Strahlungsintensität bereitgestellt wird.

Es wird weiterhin eine Güteinformation für die Funktion der Partikelerzeugungsvorrichtung, beispielsweise des Gemäß einer weiteren Ausführungsform wird eine GüteinformatiSynchrotrons, des Linearbeschleunigers oder des Zyklotrons bestimmt. Die Güteinformation wird automatisch in Abhängigkeit von der erfassten Partikelstrahlintensität und der aktuellen Beeinflussung der Partikelstrahlintensität bestimmt. Wenn sich beispielsweise die im Beschleuniger gespeicherte Teilchenzahl aufgrund von technischen Problemen mit der Zeit verändert, muss bei einem Synchrotron mit Linearbeschleuniger die Einstellung des Anteils der ausgekoppelten Bestrahlungspartikel entsprechend nachgestellt werden, um den vorgegebenen Sollwert für die Partikelstrahlintensität einzuhalten. Durch geeignete Protokollierung und Bewertung der Einstellung des Anteils der ausgekoppelten Bestrahlungspartikel kann somit eine Güteinformation der Partikelerzeugungsvorrichtung abgeleitet werden. Auf Basis dieser Güteinformation können beispielsweise Maßnahmen zur vorbeugenden Wartung der Partikelerzeugungsvorrichtung abgeleitet werden.

Gemäß der vorliegenden Erfindung wird eine Partikeltherapieanlage bereitgestellt. Die Partikeltherapieanlage umfasst eine Partikelerzeugungsvorrichtung zum Erzeugen und Beschleunigen von Bestrahlungspartikeln, eine Strahlerzeugungsvorrichtung, eine Erfassungsvorrichtung, eine Rastersteuerung und eine Partikelstrahlbeeinflussungsvorrichtung. Die Strahlerzeugungsvorrichtung, beispielsweise ein Knock-Out-Exciter, ist derart ausgestaltet, dass sie aus zumindest einem Anteil der Bestrahlungspartikel der Partikelerzeugungsvorrichtung einen Partikelstrahl erzeugt. Die Erfassungsvorrichtung ist in der Lage, automatisch eine Partikelstrahlintensität des Partikelstrahls zu erfassen. Die Partikelstrahlbeeinflussungsvorrichtung ist derart ausgestaltet, dass sie die Partikelstrahlintensität in Abhängigkeit von der erfassten Partikelstrahlintensität und einem vorgegebenen Sollwert für die Partikelstrahlintensität beeinflusst und somit einstellt. Die Rastersteuerung ist ausgestaltet, mehrere Rasterpunkte mit dem Partikelstrahl gemäß einem vorgegebenen Bestrahlungsplan sequentiell zu bestrahlen. Der Bestrahlungsplan ordnet jedem der mehreren Rasterpunkte jeweils eine Strahlen dosis zu. In Abhängig Keit von der Strahler dosis wird jeweils Sollwert für die Partikelstrahlintensität zu geordnet. Die Rastersteuerung ist mit der Partikelstrahlbeeinflussungsvorrichtung gekoppelt und überträgt den Sollwert eines zu bestrahlenden Rasterpunkts zu der Partikelstrahlbeeinflussungsvorrichtung. Die Partikelstrahlbeeinflussungsvorrichtung verwendet den Sollwert, um die gewünschte Partikelstrahlintensität für den zu bestrahlenden Rasterpunkt einzustellen. Dadurch können sowohl kleine als auch große Bestrahlungsdosen für verschiedene Rasterpunkte realisiert und in kurzen Bestrahlungszeiten bereitgestellt werden. Dadurch kann die Nutzung der Partikeltherapieanlage verbessert werden und die Behandlungszeit für den Patienten verkürzt werden.

Die Partikeltherapieanlage kann weiterhin eine Partikelstrahlzuführungseinheit umfassen, welche den Partikelstrahl zu einem Partikelstrahlausgang eines Behandlungsplatzes leitet. In diesem Fall ist die Erfassungsvorrichtung zum automatischen Erfassen der Partikelstrahlintensität vorzugsweise an dem Partikelstrahlausgang angeordnet. Dadurch kann eine genaue und gewünschte Strahlintensität an dem Partikelstrahlausgang sichergestellt werden.

Die Partikeltherapieanlage kann weiterhin eine Partikelstrahlzuführungseinheit umfassen, welche wahlweise den Partikelstrahl zu einem von mehreren Behandlungsplätzen leitet. An jedem Behandlungsplatz ist eine Erfassungsvorrichtung zum automatischen Erfassen der Partikelstrahlintensität des ausgeleiteten Partikelstrahls, insbesondere zum Erfassen der applizierten Partikelstrahlintensität, vorgesehen. Die Partikeltherapieanlage kann weiterhin eine Umschalteinheit umfassen, welche die erfasste Partikelstrahlintensität desjenigen Behandlungsplatzes zu der Partikelstrahlbeeinflussungsvorrichtung leitet, zu welchem der Partikelstrahl aktuell geleitet wird. Dadurch können mehrere Behandlungsplätze der Reihe nach mit einem Partikelstrahl versorgt werden, wodurch die Partikeltherapieanlage effizienter genutzt werden kann. Mit Hilfe der Umschalteinheit wird sichergestellt, dass an jedem Behandlungsplatz die gewünschte Partikelstrahlintensität bereitgestellt wird.

Gemäß der Erfindung umfasst die Partikeltherapieanlage eine Gütebestimmungsvorrichtung zum Bestimmen einer Güteinformation für dei Funktion der Partikelerzeugungsvorrichtung. Die Güteinformation wird von der Gütebestimmungsvorrichtung in Abhängigkeit von der erfassten Partikelstrahlintensität und der Beeinflussung der Partikelstrahlintensität durch die Partikelstrahlbeeinflussungsvorrichtung bestimmt. Dadurch können Störungen oder zukünftig erforderliche Wartungen an der Partikeltherapieanlage frühzeitig erkannt und behoben werden.

Die zuvor beschriebene Partikeltherapieanlage ist weiterhin zur Durchführung des zuvor beschriebenen Verfahrens geeignet und umfasst daher die im Zusammenhang mit dem Verfahren beschriebenen Vorteile.

Auch offenbart ist ein Computerprogrammprodukt, insbesondere eine Software, welche in einem Speicher einer programmierbaren Steuerung, beispielsweise der Partikelstrahlbeeinflussungsvorrichtung, der zuvor beschriebenen Partikeltherapieanlage geladen werden kann. Mit Programmmitteln dieses Computerprogrammprodukts können alle zuvor beschriebenen Varianten des Verfahrens ausgeführt werden, wenn das Computerprogrammprodukt in der Partikeltherapieanlage ausgeführt wird.

Diesbezüglich stellt die vorliegende Erfindung einen elektronisch lesbaren Datenträger, wie zum Beispiel eine CD oder DVD, bereit, auf welchem elektronisch lesbare Steuerinformationen, insbesondere Software, gespeichert sind. Wenn diese Steuerinformationen von dem Datenträger gelesen und einer Steuervorrichtung, beispielsweise der Partikelstrahlbeeinflussungsvorrichtung, der Partikeltherapieanlage gespeichert werden, können alle Varianten des zuvor beschriebenen Verfahrens mit der Partikeltherapieanlage durchgeführt werden.

Nachfolgend wird die vorliegende Erfindung anhand bevorzugter Ausführungsformen unter Bezugnahme auf die Zeichnungen erläutert.
Fig. 1 zeigt einen gewünschten Verlauf einer Partikelstrahlintensität über der Zeit.
Fig. 2 zeigt einen typischen Verlauf einer Partikelstrahlintensität über der Zeit gemäß dem Stand der Technik.
Fig. 3 zeigt eine schematische Darstellung einer Partikeltherapieanlage gemäß einer Ausführungsform der vorliegenden Erfindung.
Fig. 4 zeigt einen zeitlichen Verlauf einer Partikelstrahlintensität gemäß einer Ausführungsform der vorliegenden Erfindung.
Fig. 5 zeigt den Verlauf einer Partikelstrahlintensität gemäß der vorliegenden Erfindung im Vergleich zu einem Verlauf der Partikelstrahlintensität gemäß dem Stand der Technik.
Fig. 6 zeigt einen Bestrahlungsplan, welcher jedem Rasterpunkt des Bestrahlungsplans eine Bestrahlungsdosis zuordnet.
Fig. 7 zeigt einen Verlauf einer Partikelstrahlintensität gemäß einer weiteren Ausführungsform der vorliegenden Erfindung.

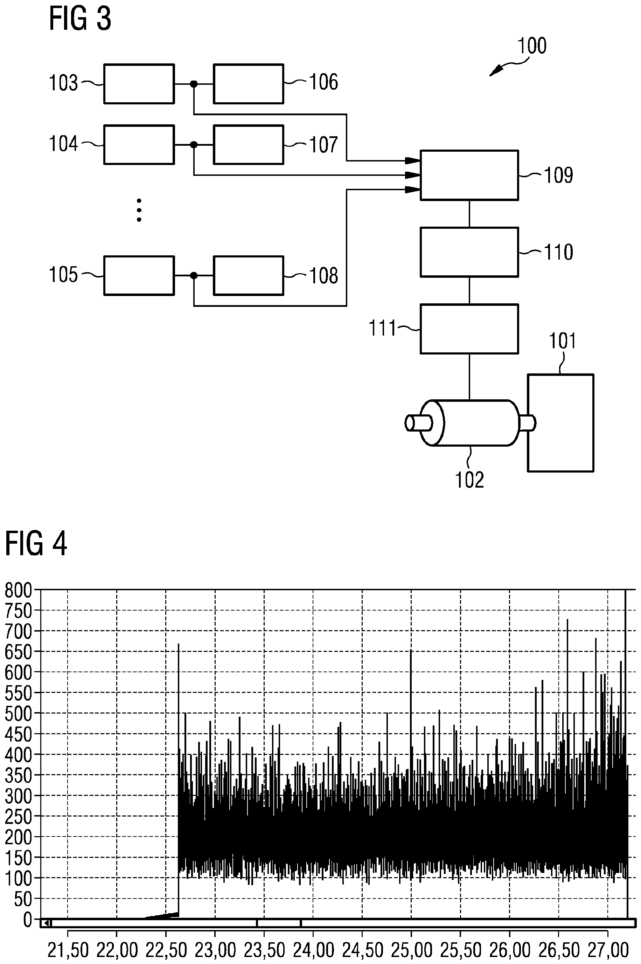

Fig. 3 zeigt eine Partikeltherapieanlage 100. Die Partikeltherapieanlage 100 umfasst eine Partikelerzeugungsvorrichtung 101, eine Strahlerzeugungsvorrichtung 102 und eine nicht gezeigte Partikelstrahlzuführungseinheit. In der Partikelerzeugungsvorrichtung 101, beispielsweise ein Linearbeschleuniger und ein Synchrotron oder ein Zyklotron, werden Bestrahlungspartikel, beispielsweise Protonen oder Kohlenstoffionen, erzeugt und beschleunigt. Bei einem Synchrotron und Linearbeschleuniger wird mit Hilfe der Strahlerzeugungsvorrichtung 102 wird ein Anteil der erzeugten und beschleunigten Partikel oder Teilchen aus der Partikelerzeugungsvorrichtung 101 ausgekoppelt und als ein Partikelstrahl in die nicht gezeigte Partikelstrahlzuführungseinheit geleitet. Die Strahlerzeugungsvorrichtung 102 kann beispielsweise ein Knock-Out-Exciter sein, welcher eine Hochfrequenz einstellbarer Leistung auf einen Teilchenstrahl in der Partikelerzeugungsvorrichtung 101 gibt. Je höher die Leistung ist, umso mehr Teilchen werden pro Zeit ausgekoppelt und dem ausgekoppelten Partikelstrahl zugeführt. Die Partikelstrahlzuführungseinheit leitet den so erzeugten Partikelstrahl wahlweise zu einem von mehreren Behandlungsplätzen, welche beispielsweise in unterschiedlichen Behandlungsräumen angeordnet sein können. Die Partikeltherapieanlage umfasst weiterhin an jedem Behandlungsplatz eine Erfassungsvorrichtung 103-105 zum Erfassen der Partikelstrahlintensität des Partikelstrahls, welcher dem Behandlungsplatz von der Partikelstrahlzuführungseinheit zugeführt wird. In der Fig. 3 sind exemplarisch drei Behandlungsplätze gezeigt, wobei an einem ersten Behandlungsplatz die Erfassungsvorrichtung 103 angeordnet ist, an einem zweiten Behandlungsplatz die Erfassungsvorrichtung 104 angeordnet ist und an einem dritten Behandlungsplatz die Erfassungsvorrichtung 105 angeordnet ist. Die Erfassungsvorrichtungen 103-105 erfassen die Strahlintensität beispielsweise mit Hilfe von gasgefüllten Ionisationskammern und darin angeordneten Plattenkondensatoren. Die so erfasste Partikelstrahlintensität gibt eine Partikelstrahldosis pro Zeiteinheit an. Jeder Erfassungsvorrichtung 103-105 ist jeweils eine Dosisbestimmungsvorrichtung 106-108 zugeordnet, welche jeweils die Partikelstrahlintensitäten der zugeordneten Erfassungsvorrichtungen über der Zeit aufintegrieren, um eine Partikelstrahldosis an dem Behandlungsplatz zu bestimmen.

Die Partikeltherapieanlage 100 umfasst weiterhin eine Umschalteinheit 109, welche mit den Erfassungsvorrichtungen 103-105 gekoppelt ist. Die Umschalteinheit 109 ist weiterhin mit einer Partikelstrahlbeeinflussungsvorrichtung 110 gekoppelt. Die Partikelstrahlbeeinflussungsvorrichtung 110 kann beispielsweise eine elektronische Steuerung, wie zum Beispiel einen Mikroprozessor, umfassen. Eine übergeordnete (nicht gezeigte) Steuerung der Partikeltherapieanlage 100, welche auch die Partikelstrahlzuführungseinheit steuert, stellt der Umschalteinheit 109 eine Information bereit, welcher Behandlungsplatz aktuell von der Partikelstrahlzuführungseinheit mit dem Partikelstrahl versorgt wird. Dementsprechend wählt die Umschalteinheit 109 die erfasste Partikelstrahlintensität von einer der Erfassungsvorrichtungen 103-105 aus, um sie zu der Partikelstrahlbeeinflussungsvorrichtung 110 weiterzuleiten. Die Verbindung zwischen den Erfassungsvorrichtungen 100-105 und der Umschalteinheit 109 kann beispielsweise digitale Signale über beispielsweise separate Leitungen oder ein Bussystem übertragen. Die übergeordnete Steuerung stellt der Partikelstrahlbeeinflussungsvorrichtung 110 einen Sollwert für die Partikelstrahlintensität bereit und die Partikelstrahlbeeinflussungsvorrichtung 110 stellt einen Steuerwert für einen Ansteuerschaltkreis 111 für die Strahlerzeugungsvorrichtung 102 bereit. Der Steuerwert für den Ansteuerschaltkreis 111 wird von der Partikelstrahlbeeinflussungsvorrichtung 110 in Abhängigkeit von der erfassten Partikelstrahlintensität von einer der Erfassungsvorrichtungen 103-105 und dem vorgegebenen Sollwert für die Partikelstrahlintensität bestimmt. Dazu umfasst die Partikelstrahlbeeinflussungsvorrichtung 110 beispielsweise einen PID-Regler. Der PID-Regler stellt den Steuerwert für den Ansteuerschaltkreis 111 bereit, wodurch beispielsweise die Hochfrequenzleistung des Knock-Out-Exciters eingestellt wird, um so die Auskopplungsrate oder Extraktionsrate der Teilchen im Synchrotron auf eine zeitlich konstante Rate, welche durch den Sollwert vorgegeben ist, zu regeln. Die Partikelstrahlbeeinflussungsvorrichtung 110 kann beispielsweise in Form einer industriell üblichen Steuerung, einer so genannten speicherprogrammierbaren Steuerung, realisiert werden und somit als ein Standardbaustein in die Partikeltherapieanlage integriert werden. Der Steuerwert, welcher von der Partikelstrahlbeeinflussungsvorrichtung 110 zu dem Ansteuerschaltkreis 111 der Strahlerzeugungsvorrichtung 102 übertragen wird, kann beispielsweise analog oder bevorzugt digital übertragen werden.

Alternativ kann die Umschalteinheit 109 auch unabhängig von einer übergeordneten Steuerung die erfasste Partikelstrahlintensität von derjenigen Erfassungsvorrichtung 103-105 zu der Partikelstrahlbeeinflussungsvorrichtung 110 weiterleiten, die an einem Behandlungsplatz angeordnet ist, welcher eine von Null verschiedene Sollintensität meldet, da ein übergeordnetes System ohnehin aus Gründen der Patientensicherheit gewährleistet, dass zu einer Zeit nur genau ein Behandlungsplatz mit einem Partikelstrahl versorgt wird.

Die Partikeltherapieanlage 100 umfasst weiterhin eine (nicht gezeigte) Gütebestimmungsvorrichtung, welche beispielsweise in der zuvor genannten übergeordneten Steuervorrichtung der Partikeltherapieanlage 100 integriert ist. Die Gütebestimmungsvorrichtung erfasst den Steuerwert, welcher von der Partikelstrahlbeeinflussungsvorrichtung 110 zu dem Ansteuerschaltkreis 111 ausgegeben wird und protokolliert die Steuerwerte im Laufe der Zeit zusammen mit den Sollwerten für die Partikelstrahlintensität. Wenn sich beispielsweise die von der Partikelerzeugungsvorrichtung 101 erzeugte und gespeicherte Partikelzahl aufgrund von beispielsweise technischen Problemen mit der Zeit verändert, nimmt der Steuerwert der Partikelstrahlbeeinflussungsvorrichtung 110 ungewöhnliche Werte an, beispielsweise obere oder untere Grenzwerte, bevor ein Ende der geplanten Bestrahlungszeit erreicht ist. Durch eine geeignete Protokollierung und Bewertung des Zeitpunkts des Erreichens des Grenzwerts kann so ein Güteparameter für die Funktion der Partikelerzeugungsvorrichtung 101 abgeleitet werden. Auf der Grundlage dieses Güteparameters können Maßnahmen zur vorbeugenden Wartung der Partikelerzeugungsvorrichtung 101 abgeleitet werden.

Wenn der Steuerwert, welcher von der Partikelstrahlbeeinflussungsvorrichtung 110 zu dem Ansteuerschaltkreis 111 ausgegeben wird, einen Grenzwert erreicht, kann dies auch signalisieren, dass sich keine Partikel mehr im Beschleuniger der Partikelerzeugungsvorrichtung 101 befinden. Diese Information kann genutzt werden, um die geregelte Anwendung des Partikelstrahls solange durchzuführen, bis durch den Steuerwert von der Partikelstrahlbeeinflussungsvorrichtung 110 zu dem Ansteuerschaltkreis 111 signalisiert wird, dass sich keine Partikel mehr im Beschleuniger befinden. Damit kann erreicht werden, dass die im Beschleuniger gespeicherte Partikelzahl optimal ausgenutzt wird. Dies kann zu einer verringerten Umweltbelastung beitragen, da der gespeicherte und beschleunigte Reststrahl anderenfalls vernichtet werden müsste, was zu einer radioaktiven Kontaminierung einer Vernichtungseinheit und/oder von Anlagenteilen führen würde.

Da die Information der Partikelstrahlintensität von den Erfassungsvorrichtungen oder Detektoren 103-105 in Echtzeit zur Regelung der Partikelstrahlintensität zu der Partikelstrahlbeeinflussungsvorrichtung 110 ausgeleitet wird, kann eine erheblich genauere Partikelstrahlintensität an den Behandlungsplätzen bereitgestellt werden. Fig. 4 zeigt den Verlauf einer gemäß der vorliegenden Erfindung geregelten Partikelstrahlintensität. Die Intensität wurde ebenso wie in Fig. 2 im Abstand von 50 µs erfasst. Die Regelung wurde zum Zeitpunkt t=22,7 s zugeschaltet. Im Vergleich mit der Fig. 2 ist deutlich zu erkennen, dass die langfristigen Fluktuationen durch die Regelung deutlich verringert werden konnten.

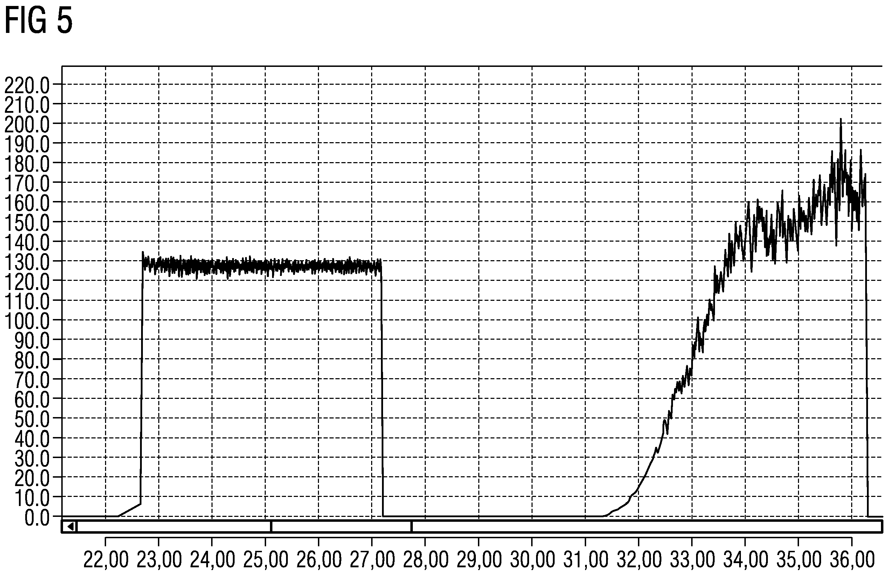

Fig. 5 zeigt einen Vergleich der Partikelstrahlintensität mit und ohne Regelung. In Fig. 5 wurden die Intensitätsdaten mit einem FIR-Filter durch Mittelung über 20 ms geglättet. Wie aus der Fig. 5 ersichtlich ist, sind in dem geregelten Partikelstrahl in dem Zeitbereich von näherungsweise 22,7 s bis 27,2 s nicht nur die langsamen Fluktuationen deutlich verringert, sondern auch die kurzzeitigen. In dem ungeregelten Intensitätsverlauf von näherungsweise 31,4 s bis 36,3 s sind neben den erheblich stärkeren langsamen Fluktuationen auch die deutlich stärkeren kurzfristigen Fluktuationen zu erkennen.

Tumore werden bei einer Partikelstrahltherapie typischerweise im Rasterscanverfahren behandelt. Dabei wird der Tumor logisch in eine Vielzahl von Rastervolumina, so genannten Rasterpunkten, aufgeteilt und in einem Bestrahlungsplan jedem dieser Rasterpunkte eine Bestrahlungsdosis zugeordnet. Zur Bestrahlung eines Rasterpunkts wird der Partikelstrahl auf den Rasterpunkt gerichtet und Partikel mit einer vorbestimmten Energie, welche die Eindringtiefe des Partikels bestimmt, in den Tumor ausgestrahlt. Der Behandlungsplan kann dabei stark unterschiedliche Partikeldosen für jeden Rasterpunkt aufweisen.

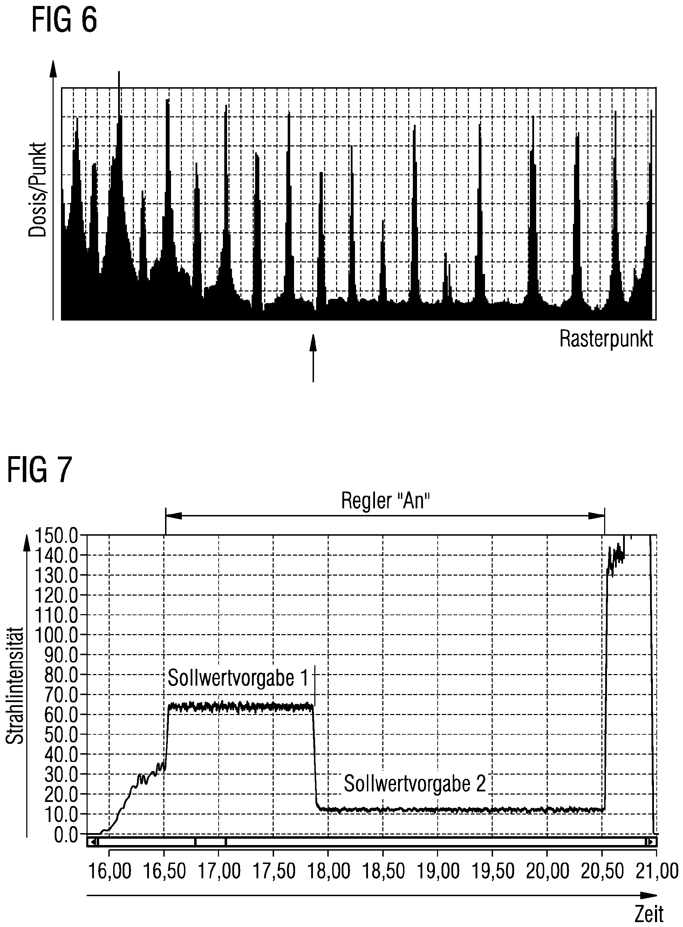

Fig. 6 zeigt beispielhaft die gewünschten Partikeldosen für eine Vielzahl von Rasterpunkten eines Bestrahlungsplans. Üblicherweise ist eine minimale Zeit pro Rasterpunkt während der Anwendung der Bestrahlung des Rasterpunkts notwendig. Diese minimale Zeit für die Bestrahlung des Rasterpunkts mit minimaler Dosis beschränkt die maximale Strahlintensität, die verwendet werden kann. In Fig. 6 ist der Rasterpunkt mit minimaler Dosis mit einem Pfeil gekennzeichnet. Bei einer Partikeltherapieanlage gemäß dem Stand der Technik, bei der die Partikelstrahlintensität nicht wie zuvor beschrieben geregelt wird, wird für viele Rasterpunkte mit deutlich höherer Dosis daher eine verhältnismäßig lange Bestrahlungszeit in Kauf genommen, die durch die Dosis des Rasterpunkts geringster Dosis dominiert wird.

Mit der in Fig. 3 gezeigten Partikeltherapieanlage 100 mit einer geregelten Partikelstrahlintensität kann hingegen die Partikelstrahlintensität von Rasterpunkt zu Rasterpunkt dynamisch verändert werden. Der Sollwert für die Strahlintensität kann beispielsweise aus dem Bestrahlungsplan automatisch abgeleitet werden und in Echtzeit während der Bestrahlung der verschiedenen Rasterpunkte zu der Partikelstrahlbeeinflussungsvorrichtung 110 synchronisiert übertragen werden. Da das Beschleunigersystem die Eigenschaft haben kann, auf eine Stellgrößenänderung des Reglers mit einer Verzögerungszeit von einigen Millisekunden zu reagieren, kann die Vorgabe des Sollwerts für die Partikelstrahlbeeinflussungsvorrichtung 110 mit einer "Vorlaufzeit" ausgegeben werden. Wenn bei dem übergeordneten Steuersystem vor Beginn der Bestrahlung eine zeitlich geordnete Liste mit den Daten der Rasterpunkte vorliegt (Bestrahlungsplan), kann in einer Vorverarbeitungsstufe durch einen geeigneten Algorithmus der optimale Zeitpunkt der Sollwertübergabe an die Partikelstrahlbeeinflussungsvorrichtung 110 bestimmt werden. Auf diese Weise kann die Strahlintensität immer optimal an die Anforderungen des jeweiligen Rasterpunkts angepasst werden.

Fig. 7 zeigt eine Änderung der Strahlungsintensität aufgrund einer Änderung eines vorgegebenen Sollwerts.

Ein Vorteil der vorliegenden Erfindung ergibt sich daraus, dass die Strahlintensität nicht mehr durch den Rasterpunkt niedrigster Dosis beschränkt ist. Durch die Veränderung der Partikelstrahlintensität können Rasterpunkte, für die eine hohe Strahlendosis vorgesehen ist, in erheblich kürzerer Zeit mit der vorgesehenen Dosis bestrahlt werden. Dadurch kann die gesamte Bestrahlungszeit erheblich verringert werden. Weiterhin können die minimalen Dosen pro Rasterpunkt in den Therapieplanungssystemen deutlich geringer angesetzt werden. Dadurch wird eine verbesserte Homogenität und Konformität der Bestrahlung erreicht. Gleichzeitig wird eine lange Bestrahlungszeit durch die aktive Partikelstromintensitätsregelung vermieden. Damit wird der Patientendurchsatz einer Partikeltherapieanlage deutlich erhöht und die Qualität der Behandlungspläne verbessert.

### Bezugszeichenliste

- 100: Partikeltherapieanlage
- 101: Partikelerzeugungsvorrichtung
- 102: Strahlerzeugungsvorrichtung
- 103: Erfassungsvorrichtung
- 104: Erfassungsvorrichtung
- 105: Erfassungsvorrichtung
- 106: Dosisbestimmungsvorrichtung
- 107: Dosisbestimmungsvorrichtung
- 108: Dosisbestimmungsvorrichtung
- 109: Umschalteinheit
- 110: Partikelstrahlbeeinflussungsvorrichtung
- 111: Ansteuerschaltkreis

## Patentansprüche

1. Partikeltherapieanlage, umfassend:
eine Partikelerzeugungsvorrichtung (101) zum Erzeugen und Beschleunigen von Bestrahlungspartikeln,
eine Strahlerzeugungsvorrichtung (102) zum Erzeugen eines Partikelstrahls aus zumindest einem Anteil der erzeugten Bestrahlungspartikel,
eine Erfassungsvorrichtung (103-105) zum automatisches Erfassen einer Partikelstrahlintensität des Partikelstrahls,
eine Rastersteuerung, welche ausgestaltet ist, Rasterpunkte mit dem Partikelstrahl gemäß einem vorgegebenen Bestrahlungsplan sequentiell zu bestrahlen, wobei der Bestrahlungsplan jedem der Rasterpunkte jeweils einen Sollwert für die Partikelstrahlintensität zuordnet, und
eine Partikelstrahlbeeinflussungsvorrichtung (110), welche mit der Rastersteuerung gekoppelt ist und ausgestaltet ist, die Partikelstrahlintensität in Abhängigkeit von der erfassten Partikelstrahlintensität und dem jeweils vorgegebenen Sollwert für die Partikelstrahlintensität des zu bestrahlenden Rasterpunkts zu beeinflussen,
**gekennzeichnet durch**
eine Gütebestimmungsvorrichtung zum Bestimmen einer Güteinformation der Partikelerzeugungsvorrichtung, wobei
die Gütebestimmungsvorrichtung dazu ausgelegt ist:
einen Steuerwert, welcher von der Partikelstrahlbeeinflussungsvorrichtung (110) zu einem Ansteuerschaltkreis (111) zum Beeinflussen der Strahlerzeugungsvorrichtung (102) ausgegeben wird, zu erfassen,
die im Laufe der Zeit erfassten Steuerwerte zusammen mit den Sollwerten für die Partikelstrahlintensität zu protokollieren, und
durch Bewertung eines Zeitpunkts des Erreichens eines Grenzwerts der erfassten Steuerwerte einen Güteparameter für die Funktion der Partikelerzeugungsvorrichtung (101) abzuleiten.

2. Partikeltherapieanlage nach Anspruch 1, ferner umfassend eine Partikelstrahlzuführungseinheit zum Leiten des Partikelstrahls zu einem Partikelstrahlausgang eines Behandlungsplatzes, wobei die Erfassungsvorrichtung (103-105) an dem Partikelstrahlausgang angeordnet ist.

3. Partikeltherapieanlage nach einem der vorhergehenden Ansprüche, wobei der Sollwert der Partikelstrahlintensität einen zeitlich nicht konstanten Verlauf aufweist.

4. Partikeltherapieanlage nach einem der vorhergehenden Ansprüche, wobei die Partikelstrahlbeeinflussungsvorrichtung (110) einen Proportionalregler, einen integrierenden Regler, einen differenzierenden Regler oder eine Kombination daraus umfasst.

5. Partikeltherapieanlage nach einem der vorhergehenden Ansprüche, wobei die Partikelerzeugungsvorrichtung (101) ein Synchrotron, einen Linearbeschleuniger und/oder ein Zyklotron umfasst.

6. Partikeltherapieanlage nach einem der vorhergehenden Ansprüche, ferner umfassend:
eine Partikelstrahlzuführungseinheit zum wahlweisen Leiten des Partikelstrahls zu einem von mehreren Behandlungsplätzen, wobei an jedem Behandlungsplatz eine Erfassungsvorrichtung (103-105) zum automatisches Erfassen der Partikelstrahlintensität des ausgeleiteten Partikelstrahls vorgesehen ist, und
eine Umschalteinheit (109), welche ausgestaltet ist, die erfasste Partikelstrahlintensität desjenigen Behandlungsplatzes zu der Partikelstrahlbeeinflussungsvorrichtung (110) zu leiten, zu welchem der Partikelstrahl geleitet wird.

7. Elektronisch lesbarer Datenträger mit darauf gespeicherten elektronisch lesbaren Steuerinformationen, welche derart ausgestaltet sind, dass bei Verwendung des Datenträgers in einer Partikelstrahlbeeinflussungsvorrichtung (110), diese das nachfolgende Verfahren zum Betreiben einer Partikeltherapieanlage nach einem der vorhergehenden Ansprüche durchführt, das umfasst:
Erzeugen und Beschleunigen von Bestrahlungspartikeln,
Erzeugen eines Partikelstrahls aus zumindest einem Anteil der erzeugten Bestrahlungspartikel,
automatisches Erfassen einer Partikelstrahlintensität des Partikelstrahls,
automatisches sequentielles Bestrahlen mehrerer Rasterpunkte mit dem Partikelstrahl gemäß einem vorgegebenen Bestrahlungsplan, wobei der Bestrahlungsplan jedem der mehreren Rasterpunkte jeweils einen Sollwert für die Partikelstrahlintensität zuordnet, und
automatisches Beeinflussen der Partikelstrahlintensität in Abhängigkeit von der erfassten Partikelstrahlintensität und dem jeweiligen Sollwert des zu bestrahlenden Rasterpunkts,
**dadurch gekennzeichnet, dass**
zum Bestimmen einer Güteinformation der Partikelerzeugungsvorrichtung:
ein Steuerwert, welcher von der Partikelstrahlbeeinflussungsvorrichtung (110) zu einem Ansteuerschaltkreis (111) zum Beeinflussen der Strahlerzeugungsvorrichtung (110) ausgegeben wird, erfasst wird,
die im Laufe der Zeit erfassten Steuerwerte zusammen mit den Sollwerten für die Partikelstrahlintensität protokolliert werden, und
durch Bewertung eines Zeitpunkts des Erreichens eines Grenzwerts der erfassten Steuerwerte ein Güteparameter für die Funktion der Partikelerzeugungsvorrichtung (101) abgeleitet wird.

## Claims

1. A particle therapy system comprising:
a particle generating apparatus (101) for generating and accelerating irradiation particles;
a beam generating apparatus (102) for generating a particle beam from at least a portion of the generated irradiation particles;
a detection apparatus (103-105) for an automatic detection of a particle beam intensity of the particle beam;
a grid control that is configured to sequentially irradiate grid points with the particle beam in accordance with a predefined irradiation plan, with the irradiation plan associating a respective desired value for the particle beam intensity to each of the grid points; and
a particle beam influencing apparatus (110) that is coupled to the grid control and that is configured to influence the particle beam intensity in dependence on the detected particle beam intensity and on the respective predefined desired value for the particle beam intensity of the grid point to be irradiated,
**characterized by**
a quality determination apparatus for determining a piece of quality information of the particle generating apparatus, with
the quality determination apparatus being configured:
to detect a control value that is output by the particle beam influencing apparatus (110) to a control circuit (111) for influencing the beam generating apparatus (102);
to record the control values detected over the course of time together with the desired values for the particle beam intensity; and
to derive a quality parameter for the function of the particle generating apparatus (101) by evaluating a point of time of the reaching of a limit value of the detected control values.

2. A particle therapy system in accordance with claim 1, further comprising a particle beam supply unit for conducting the particle beam to a particle beam outlet of a treatment station, wherein the detection apparatus (103-105) is arranged at the particle beam outlet.

3. A particle therapy system in accordance with one of the preceding claims, wherein the desired value of the particle beam intensity has a progression not constant in time.

4. A particle therapy system in accordance with one of the preceding claims, wherein the particle beam influencing apparatus (110) comprises a proportional regulator, an integrating regulator, a differentiating regulator, or a combination thereof.

5. A particle therapy system in accordance with one of the preceding claims, wherein the particle generating apparatus (101) comprises a synchotron, a linear accelerator and/or a cyclotron.

6. A particle therapy system in accordance with one of the preceding claims further comprising:
a particle beam supply unit for the selective conducting of the particle beam to one of a plurality of treatment stations, wherein a detection apparatus (103-105) for an automatic detection of the particle beam intensity of the outwardly conducted particle beam is provided at each treatment station; and
a switching unit (109) that is configured to conduct the detected particle beam intensity of that treatment station to the particle beam influencing apparatus (110) to which the particle beam is conducted.

7. An electronically readable data carrier having electronically readable control information that is stored thereon and this is configured such that on the use of the data carrier in a particle beam influencing apparatus (110), it carries out the following method of operating a particle therapy system in accordance with one of the preceding claims that comprises:
generating and accelerating irradiation particles;
generating a particle beam from at least one portion of the generated irradiation particles;
automatically detecting a particle beam intensity of the particle beam;
automatically sequentially irradiating a plurality of grid points with the particle beam in accordance with a predefined irradiation plan, wherein the irradiation plan associates a respective desired value for the particle beam intensity to each of the plurality of grid points; and
automatically influencing the particle beam intensity in dependence on the detected particle beam intensity and on the respective desired value of the grid point to be irradiated,
**characterized in that,**
to determine a piece of quality information of the particle generating apparatus:
a control value that is output by the particle beam influencing apparatus (110) to a control circuit (111) for influencing the beam generating apparatus (110) is detected,
the control values detected over the course of time together with the desired values for the particle beam intensity are recorded; and
a quality parameter for the function of the particle generating apparatus (101) is derived by evaluating a point of time of the reaching of a limit value of the detected control values.

## Revendications

1. Installation de thérapie à particules, comprenant :
un dispositif de génération de particules (101) destiné à générer et accélérer des particules d'irradiation,
un dispositif de génération de faisceau (102) destiné à générer un faisceau de particules à partir d'au moins une partie des particules d'irradiation générées,
un dispositif de détection (103-105) destiné à détecter automatiquement une intensité de faisceau de particules du faisceau de particules,
une commande à quadrillage, qui est conçue pour irradier de manière séquentielle des points du quadrillage avec le faisceau de particules selon un plan d'irradiation prédéfini, le plan d'irradiation associant à chacun des points du quadrillage respectivement une valeur de consigne pour l'intensité de faisceau de particules, et
un dispositif d'influence sur le faisceau de particules (110), qui est couplé à la commande à quadrillage et est conçu pour influer sur l'intensité du faisceau de particules en fonction de l'intensité de faisceau de particules détectée et de la valeur de consigne prédéfinie respective pour l'intensité de faisceau de particules du point du quadrillage à irradier,
**caractérisée par**
un dispositif de détermination de la qualité destiné à déterminer une information de qualité du dispositif de génération de particules,
le dispositif de détermination de la qualité étant conçu pour :
détecter une valeur de commande, qui est émise par le dispositif d'influence sur le faisceau de particules (110) vers un circuit de commande (111) afin d'influer sur le dispositif de génération de faisceau (102),
consigner les valeurs de commande détectées au fil du temps conjointement avec les valeurs de consigne pour l'intensité de faisceau de particules, et
dériver un paramètre de qualité pour le fonctionnement du dispositif de génération de particules (101) en évaluant un moment de l'atteinte d'une valeur limite des valeurs de commande détectées.

2. Installation de thérapie à particules selon la revendication 1, comprenant en outre une unité d'acheminement de faisceau de particules destinée à guider le faisceau de particules vers une sortie de faisceau de particules d'un emplacement de traitement, le dispositif de détection (103-105) étant disposé à la sortie de faisceau de particules.

3. Installation de thérapie à particules selon l'une des revendications précédentes, dans laquelle la valeur de consigne de l'intensité de faisceau de particules présente une évolution non constante dans le temps.

4. Installation de thérapie à particules selon l'une des revendications précédentes, dans laquelle le dispositif d'influence sur le faisceau de particules (110) comprend un régulateur proportionnel, un régulateur intégral, un régulateur différentiel ou une combinaison de ceux-ci.

5. Installation de thérapie à particules selon l'une des revendications précédentes, dans laquelle le dispositif de génération de particules (101) comprend un synchrotron, un accélérateur linéaire et/ou un cyclotron.

6. Installation de thérapie à particules selon l'une des revendications précédentes, comprenant en outre :
une unité d'acheminement de faisceau de particules destinée à guider de manière sélective le faisceau de particules vers un emplacement de traitement parmi plusieurs, un dispositif de détection (103-105) étant prévu à chaque emplacement de traitement pour la détection automatique de l'intensité de faisceau de particules du faisceau de particules sortant, et
une unité de commutation (109), qui est conçue pour transmettre au dispositif d'influence sur le faisceau de particules (110) l'intensité de faisceau de particules détectée de l'emplacement de traitement auquel le faisceau de particules est acheminé.

7. Support de données lisible de manière électronique, sur lequel des informations de commande lisibles de manière électronique sont enregistrées, qui sont conçues de telle manière que, lors de l'utilisation du support de données dans un dispositif d'influence sur le faisceau de particules (110), celui-ci exécute le procédé suivant de fonctionnement d'une installation de thérapie à particules selon l'une des revendications précédentes, ledit procédé comprenant :
la génération et l'accélération de particules d'irradiation,
la génération d'un faisceau de particules à partir d'au moins une partie des particules d'irradiation générées,
la détection automatique d'une intensité de faisceau de particules du faisceau de particules,
l'irradiation séquentielle automatique de plusieurs points de quadrillage avec le faisceau de particules selon un plan d'irradiation prédéfini, le plan d'irradiation associant à chacun des plusieurs points de quadrillage respectivement une valeur de consigne pour l'intensité de faisceau de particules, et
l'influence automatique sur l'intensité de faisceau de particules en fonction de l'intensité de faisceau de particules détectée et de la valeur de consigne respective du point de quadrillage à irradier,
**caractérisé en ce que**
pour déterminer une information de qualité du dispositif de génération de particules :
une valeur de commande, qui est émise par le dispositif d'influence sur le faisceau de particules (110) vers un circuit de commande (111) afin d'influer sur le dispositif de génération de faisceau (110) est détectée,
les valeurs de commande détectées au fil du temps sont consignées conjointement avec les valeurs de consigne pour l'intensité de faisceau de particules, et
en évaluant un moment de l'atteinte d'une valeur limite des valeurs de commande détectées, un paramètre de qualité pour le fonctionnement du dispositif de génération de particules (101) est dérivé.
